Europäisches Patentamt

European Patent Office

Office européen des brevets

(19)

(11) Publication number : **0 458 391 B1**

(12) # EUROPEAN PATENT SPECIFICATION

(45) Date of publication of patent specification :
09.08.95 Bulletin 95/32

(51) Int. Cl.⁶ : **G01N 11/02,** G01N 33/28,
E21B 49/00

(21) Application number : 91201143.4

(22) Date of filing : 10.05.91

(54) Pipe rheometer.

(30) Priority : 23.05.90 GB 9011501

(43) Date of publication of application :
27.11.91 Bulletin 91/48

(45) Publication of the grant of the patent :
09.08.95 Bulletin 95/32

(84) Designated Contracting States :
DE DK FR IT NL

(56) References cited :
EP-A- 0 083 524
EP-A- 0 211 112
EP-A- 0 302 558
GB-A- 2 077 367
US-A- 2 986 925
US-A- 4 510 800
US-A- 4 856 969

(73) Proprietor : SERVICES PETROLIERS
SCHLUMBERGER
42, rue Saint-Dominique
F-75007 Paris (FR)

(72) Inventor : Meeten, Gerald Henry
South Cottage,
Bromley Lane
Standon, Ware, Hertfordshire SG11 1NW (GB)

(74) Representative : Hyden, Martin Douglas
c/o Schlumberger Cambridge Research
Limited,
High Cross,
PO Box 153
Cambridge CB3 OHG (GB)

EP 0 458 391 B1

## Description

The present invention relates to a pipe rheometer to measure the rheological properties of a liquid substance, mainly its viscosity and yield stress. The invention is particularly suitable for measuring the rheological properties of the liquids in use, such as the drilling fluids used when drilling oil, gas or geothermal wells.

When drilling a well with a drillstring having a drill bit attached at its lower end, a liquid substance, known as "drilling mud", is circulated from a mud pit at the surface into the drillstring down through the drill bit and then upward in the annulus formed between the drillstring and the borehole back to the surface in the mud pit. The drilling mud has several purposes, among them to create a predetermined hydrostatic pressure in front of the formation being drilled and to lift the drilled cuttings from the drill bit area to the surface where they are separated from the mud. Additives are mixed with the mud at the surface to obtain the characteristics of the drilling mud in accordance with predetermined specifications. It is therefore important to control the drilling mud characteristics, such as its rheological properties, during the drilling operations so as to keep them within the specifications by adding or withdrawing some additives.

Other wellbore fluids are used, such as cements, spacers and completion fluids, for which the rheological properties must be controlled and adjusted if necessary.

Current techniques set by the American Petroleum Institute (API) to measure the rheological properties of the various wellbore fluids are based on the time taken for a sample to empty from a funnel of specified geometry ("Marsh funnel") or on the torque transmitted between two concentric and coaxial cylinders when one of them is rotated ("Fann rheometer" or its equivalent).

The Marsh funnel method is comparative only and does not give the shear stress-shear rate ($\tau(\gamma)$) relationship required for calculation or modelling of downhole flows. The Fann rheometer, which is the most commonly used in the oil industry, can give in principle the required relationship $\tau(\gamma)$ but in practice is subject to many sources of error and disturbances to its expected mode of operation.

Pipe rheometers are used currently in the laboratory where the environment in not harsh.

The physical principles governing the operation of the pipe rheometer are well-known. The rheology of the liquid is obtained from the laminar frictional pressure drop P across the ends of a pipe of known geometry for a volumetric flux Q. If P(Q) is the measured function, the relationship $\tau(\gamma)$ can be derived from it by suitable treatment of the data, as explained later on.

Comparisons between the Fann and other types of rheometer have shown the Fann rheometer to be good for measuring the rheological properties of Newtonian or non-Newtonian liquids. Problems occur when measuring the rheological properties of non-Newtonian liquids containing large or dense particulate materials. Most wellbore fluids used in petroleum engineering are in this category, eg drilling muds, cements and spacers.

Pipe rheometers have several advantages compared with the Fann rheometer. For example, the Fann rheometer is essentially a discrete sampler, requiring subtraction of liquid from a point in the process and delivery to the point of measurement. By comparison, pipe rheometer is intrinsically recurrent and in operation can be arranged to continuously subtract liquid, measure its rheological properties, and return the liquid to the process stream.

In addition, there is no effective means in the Fann rheometer for the resuspension of heavy particles. Progressive sedimentation out of the sensing region between the cylinders during the measurement gives time-dependent and erroneous results. In the pipe rheometer, where the liquid in the pipe is being continually replenished, there is no time-dependent sedimentation.

Furthermore, centrifugal migration of the heavy particles in the Fann rheometer causes inhomogeneous rheological properties of the liquid within the sensing zone, leading to time-dependent and erroneous results. These can be eliminated in the pipe rheometer by the proper design and conformation of the pipe.

Also, most wellbore fluids are thixotropic liquids, ie their rheological properties at a given time depend on the flow history of the sample prior to that time. Thus the rheological properties of the drilling fluids circulating in the wellbore are in general different following a period of rest. The continuous circulation of sample in the pipe rheometer more closely resembles the continuous circulation of wellbore fluids than the discrete sampling of the Fann rheometer.

In spite of its advantages over the Fann rheometer, the pipe rheometer is almost never used at the drilling site because it is a rather delicate instrument which is difficult to use in the harsh environment of a drilling rig. For example, the pipe rheometers of the prior art usually comprise strain gauges to measure the pressure drop of the liquid circulating in the pipe and a flowmeter to measure the flowrate of the liquid in the pipe. Measurements, made directly in the drilling fluids, are difficult because of the complicated structure of these liquids.

The present invention provides a pipe rheometer which is robust enough to be used in a rough environment. It does not require any pressure measurements to be made in the liquid substance whose rheological properties are to be determined.

More precisely, the present invention relates to a pipe rheometer for measuring the rheological properties of a liquid substance, the rheometer being of the type having a pipe in which said substance is circulated driven by a fluid-operated diaphragm pump having a fluid-inlet line, a fluid-outlet line, a pump input and a pump output , the pump input receiving the liquid substance and the pump output being connected to one end of the pipe, wherein the pipe's other end is maintained at a known pressure, and there is provided a gauge for measuring the pressure of said fluid operating the pump, and means for determining the flowrate of the liquid substance circulating in the pipe.

The use of fluid-operated diaphragm pumps to drive pipe rheometers is not in itself novel, and is disclosed in Mobil US-A4,510,800. However, it has not previously been appreciated that important information regarding the rheological properties of the liquid substance - such as a drilling mud - under examination can be deduced from determinations of the liquid flow rate and the pressure of the fluid driving the pump.

The known pressure is advantageously the atmospheric pressure and the means for measuring the flowrate of the liquid substance comprises means for counting the number of pump cycles during a certain period of time.

A specific embodiment of the invention will now be described by way of non-limiting example only, with reference to the accompanying drawings, wherein:
- Figure 1 is a schematic diagram of a pipe rheometer in accordance with the present invention;
- Figure 2 represents the number N of pump strokes per second versus different air pressures P, for two glycerol-water mixtures of different viscosities;
- Figure 3 represents the number N of pump strokes per second versus different air pressures P, for a waterbase drilling mud weighted with barite at different volume fractions; and
- Figure 4 shows a comparison of the yield stress data versus different concentrations of barite in a waterbase drilling mud, obtained with a pipe rheometer and a Fann rheometer.

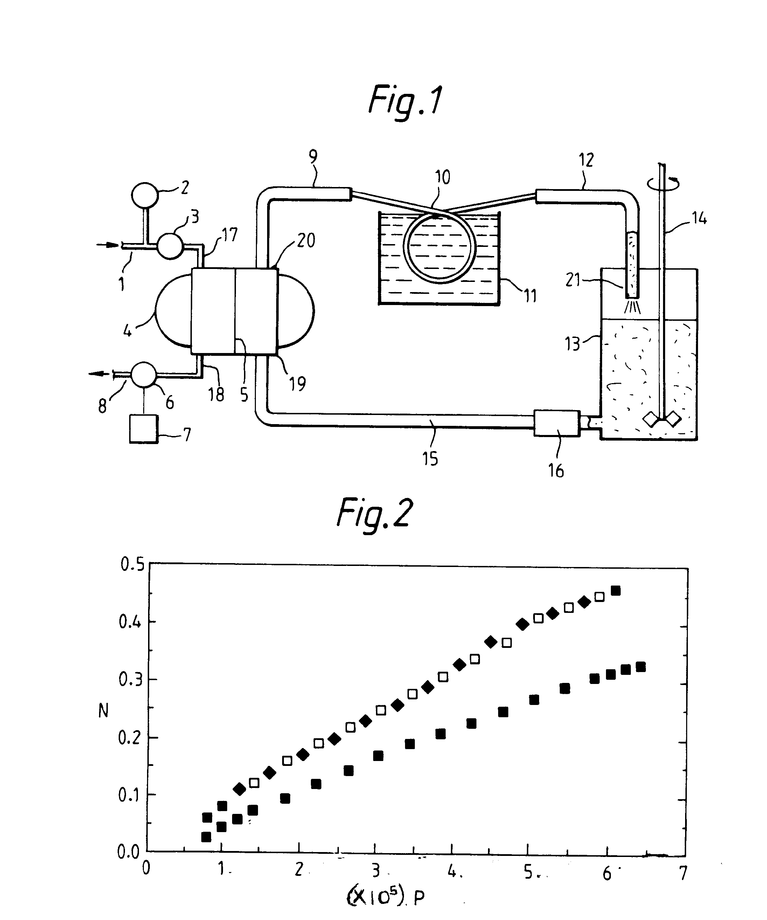

Figure 1 shows a schematic diagram of the pipe rheometer in accordance with the present invention. Compressed air is supplied via line 1 and the air pressure P relative to atmospheric pressure is set and read by the regulator gauge 2. Oiler 3 passes compressed air to a diaphragm pump 4 via a fluid-inlet line 17. The purpose of the oiler 3 is to lubricate the pump by delivering a fine spray of oil. The pump exhausts the air at a pressure very close to the atmospheric pressure via the fluid-outlet line 18, pressure switch 6, electronic timer 7 and line 8. The pressure switch 6 counts the number N of pump strokes during a time unit (one minute, for example).

The pump 4 is a fluid-operated diaphragm pump, the fluid being preferentially air. The pump has an input 19 and an output 20. The pump works on a well-known principle whereby there are two chambers which communicate with the liquid substance where rheological properties are to be measured and wherein the liquid substance and the air are separated by a flexible membrane of rubber or of similar material indicated schematically by the reference 5 in Figure 1. By a combination within the pump of pneumatic switching for the air and one-way valves for the liquid substance, the supply of compressed air alternatively sweeps the liquid substance out of each chamber, around the liquid substance circuit 9, 10, 12, 13 and 15 and back to the pump. The pump can be for example the ½ inch (or 1.27 cm) diaphragm pump manufactured by The ARO Corporation, Ohio, USA. Large-diameter line 9 connects the pump output 20 to the pipe 10 which may be cooled by a suitable water bath 11 or by convection to ambient air if the temperature rises are small. Water bath 11 may also be heated or cooled away from ambient temperature if rheology-temperature characteristics of the liquid substance are required. Pipe 10 will be generally of smaller internal diameter and longer than the other pipes 9 and 12 in the liquid circuit. This ensures that the sum of the friction pressures developed across the other pipes is negligible compared with that across pipe 10 in which the rheological properties are to be measured and for which the length and internal diameter need to be know. In the described embodiment, the internal diameters of pipes 9-12 and of pipe 10 were 12.7 mm and 4 mm respectively. The length of pipes 9-12 could be between 0 and 1 meter and the length of pipe 10 could be between 3 meters (for thick liquid substance) and 30 meters.

Pipe 12 leads the liquid substance to vessel 13. The end 21 of pipe 12 is at atmospheric pressure and is located above the level of the liquid substance in vessel 13. In the laboratory, vessel 13 would be any suitable container large enough to contain several chamber volumes of the pump, preferably agitated by mechanical stirrer 14 to ensure mixing and continued suspension of any high-gravity solids. For continuous measurement of rheological properties of the drilling mud in oilfield or similar applications, vessel 13 represents the mud pit, or one of them when several are used, or more generally the open mud return channel. Line 15 returns the liquid substance to pump input 19 from near the bottom of vessel 13, hence maximising the pick-up of high-gravity solids. To homogenise the fluid, an in-line motionless mixer 16 (for example, the one manufactured by the company Koflo) is incorporated in return line 15. The time taken to empty each chamber of the pump is determined as the time period T between consecutive pressure pulses originating from the pneumatic switch mechanism of the pump. Each pressure pulse is detected by the pressure switch 6 which passes an electrical pulse to activate the electronic timer 7. The pump characteristics being known, more especially the volume of each chamber, the volume flowrate of the liquid substance is easily obtained form the time period T, or the pump frequency N which is equal to $1/_T$.

As indicated, the liquid substance and the air are separated in the pump by the flexible membrane 5. As a result, the pressure P of the air measured with the gauge 2 is substantially equal to the pressure of the liquid substance in the pump, except for a small pressure difference which could be neglected. Calibration tests have shown that this small difference was equal to $3.5 \times 10^4 Pa(0.35)$ bar for an air pressure P of $7 \times 10^5 Pa$ (7 bars). However, by calibrating the pump, this small difference in air pressure and liquid substance pressure in the pump can be taken into account.

In accordance with the present invention, the pressure drop in the pipe 10 (the pressure drop in pipe 9 and 12 being negligible) is taken equal to the pressure at the pump output 20 (which is equal to the air pressure P) provided pipe output 21 is at the atmospheric pressure. The small pressure difference above mentioned can be taken into account, if wanted, after calibration of the pump. The pressure at the end 21 of pipe 12 is supposed to be known, if not at atmospheric pressure. The pipe rheometer does not require any pressure measurement to be made in the liquid substance; these are made only in the compressed air. A separate flowmeter is also not necessary since the volume flowrate is obtained from the pump characteristics.

On the theoretical point of view, the yield stress $\tau_y$ and the plastic viscosity $\eta$ of the liquid substance are determined from the following equations. For a Bingham plastic liquid, which is given as an example, the shear stress $\tau$ is related to the shear rate $\gamma$ by

$$\tau = \tau_y + \eta_B \gamma \quad (1)$$

where $\tau_y$ is the yield stress and $\eta_B$ is the Bingham plastic viscosity. This is the relation usually assumed for drilling muds and cements.

For Bingham liquid flow in pipe of interior radius a and length 1 the Buckingham equation (Govier and Aziz, 1972, p 196, *The Flow of Complex Mixtures in Pipes,* Krieger, Florida, USA) relates the volume flow rate Q to the pressure drop P across the pipe ends by

$$Q = \frac{\pi a^4 P}{8 l \eta_B} \left( 1 - \frac{4}{3} \left( \frac{\tau_y}{\tau} \right) + \frac{1}{3} \left( \frac{\tau_y}{\tau} \right)^4 \right) \qquad (2)$$

where $\tau$ is the shear stress at the pipe wall. For $\tau \gg \tau_y$ the $\tau^4$ term can be ignored. Using $2l\tau = aP$ and putting $Q = NV_c$ wherein $V_c$ is the volume per stroke of the pump and N is the pump frequency ($N = 1/_T$), the following equation is obtained:

$$N = \frac{\pi a^4}{8 l \eta_B V_c} P - \frac{\pi a^3 \tau_y}{3 \eta_B V_c} \quad (3)$$

which shows that a graph of N plotted with P has a slope inversely proportional to the plastic viscosity $\eta_B$ and an intercept proportional to the ratio $\tau_y/\eta_B$ of the yield stress over the plastic viscosity.

For non-Bingham fluids, such as Newtonian or "power law" liquids, equations (1) to (3) are different, but well-known. They can for example be found in the above-mentioned reference. However, the method of measuring the viscosity and the yield stress from N and P remains valid.

In use, the chamber-emptying time T is measured for different air pressures P. For any Newtonian liquid, the pump frequency N plotted with P should be linear with a zero intercept. Figure 2 shows such a plot for two Newtonian glycerol-water mixtures of two different viscosities (the larger viscosity corresponding to the lower curve), showing deviations from linearity at low pressures, but with the bulk of the data obeying the expected linearity. N is expressed in $second^{-1}$ and P in Pascals. The deviations from linearity at low pressures are attributable to mechanical friction or other imperfections of the pump. Deviations from linearity at high pressures are attributable to the onset of turbulence. The viscosity of the liquid is obtained from the slope of Figure 2 in the linear region and good agreement was found with the viscosity measured directly using a Fann rheometer.

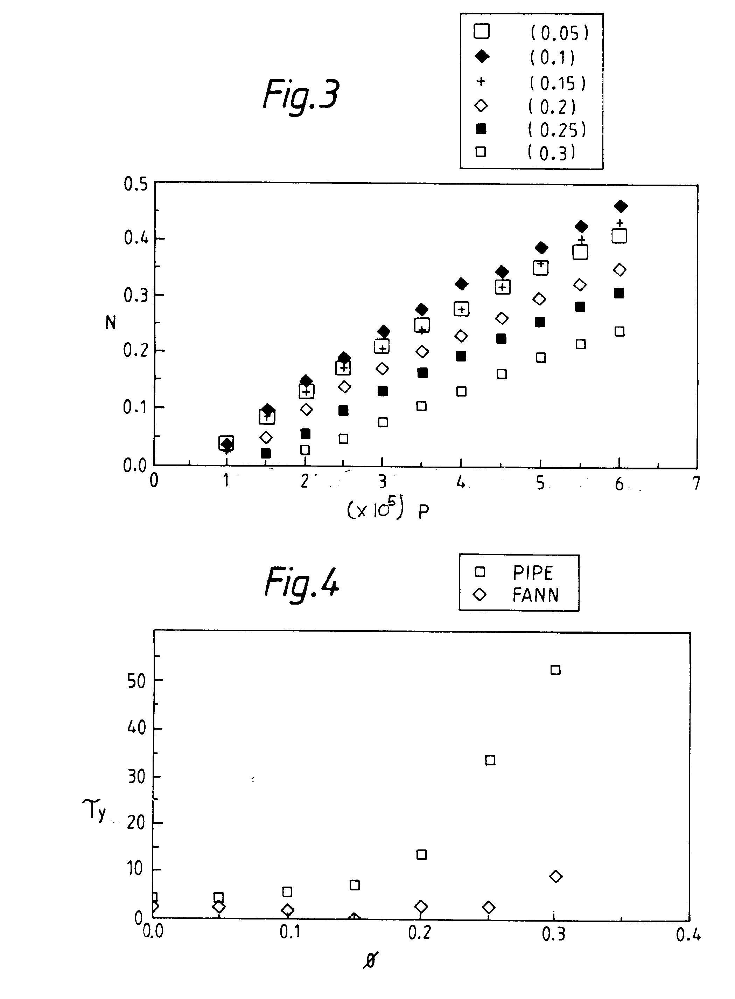

Figure 3 shows also the frequency pump N versus different air pressure P, but for a weighted waterbase drilling mud, a Bingham liquid, with six different volume fraction, from 0.05 to 0.3. The deviations from linearity at low and high pressures remain and exist for the same reasons as for the Newtonian liquid, ie as in Figure 2. A new feature is the non-zero intercept on the P axis which, in accordance with equation (3), leads to the yield stress $\tau_y$ of the mud. The slopes of the linear portions of Figure 3 also give the plastic viscosity of the mud.

Figure 4 shows a comparison of the yield stress $\tau_y$ measured for different volume fractions $\phi$ of the mud-weighting agent barite using both the 6-speed Fann rheometer and the pipe rheometer. Whereas the Fann data shows decreases in yield stress with increasing barite volume fraction $\phi$ (which is believed to be erroneous), the pipe rheometer shows that $\tau_y$ increases with $\phi$, as expected on any reasonable physical grounds.

The rheometer of the present invention, which can be used either as a laboratory instrument or kept running to continuously interrogate mud rheology during drilling, is driven by compressed air which is intrinsically safe in hazardous environments. Also, by maintaining a continuous circulation of the mud, the measured rheology is close to that which is defined by similar continuous circulation conditions in the wellbore. In addition, the rheometer overcomes sedimentation of particles, eg by mixing in the pump, by a stirrer or by mixing sections in the flow line, and rheologies of fluids having particles up to about 2 mm in size can be measured.

## Claims

1. A pipe rheometer for measuring the rheological properties of a liquid substance, the rheometer being of the type having a pipe (10) in which said substance is circulated driven by a fluid-operated diaphragm pump (4) having a fluid-inlet line (17), a fluid-outlet line (18), a pump input (19) and a pump output (20), the pump input receiving the liquid substance and the pump output being connected to one end of the pipe (10), <u>characterised in that</u> the pipe's other end (21) is maintained at a known pressure, and there is provided a gauge (2) for measuring the pressure of the fluid operating the pump (4), and means (6,7) for determining the flowrate of the liquid substance circulating in the pipe (10).

2. The pipe rheometer of claim 1, wherein said known pressure is the atmospheric pressure.

3. The pipe rheometer of claim 1 or 2, further comprising a thermostatic bath (11) in which at least part of the pipe (10) is enclosed so as to keep the fluid in the pipe within a predetermined range of temperature.

4. The pipe rheometer of any of claims 1 to 3, wherein the means (6,7) for measuring the flowrate of the liquid substance comprises means for counting the number of pump cycles during a certain period of time.

5. The pipe rheometer of claim 4, wherein the counting means are a pressure switch (6) located in the fluid-outlet line (18) and a timer (7) connected to the pressure switch.

6. The pipe rheometer of any of the preceding claims, wherein the pressure gauge (2) is in the fluid-inlet line (17).

7. The pipe rheometer of any of the preceding claims, further comprising fluid pressure-regulation means (2) in the fluid-inlet line (17).

8. The pipe rheometer of any of the preceding claims, wherein the liquid substance is contained in a tank (13) and the other end (21) of said pipe (10) is located above the surface of the liquid substance in the tank, further comprising a return line (15) connecting the tank with the pump input (19).

9. The pipe rheometer of claim 8, further comprising a mixer (16) in the return line.

10. The pipe rheometer of claim 8 or 9, further comprising a stirrer (14) in the tank.

11. The pipe rheometer of any of the preceding claims, further comprising a first connecting line (9) interjoined between the pump output (20) and said one end of the pipe (10) and/or a second connecting line (12) fixed at said other end of the pipe (10), the geometry of said connecting lines being such that the pressure drop of the liquid substance circulating in there is small compared with the pressure drop of the liquid substance circulating in the pipe.

12. The pipe rheometer of any of the preceding claims, wherein the pressure gauge (2) is calibrated so as to account for the slight difference between the pressure measured in the fluid-inlet line (17) and the pressure of the liquid substance at the pump output (20).

13. The pipe rheometer of any of the preceding claims, wherein the fluid is compressed air.

14. A method of determining the rheological properties of a drilling fluid, <u>characterised in that</u> there is employed a pipe rheometer as claimed in any of the preceding claims to measure the fluid gauge pressure P and the pump frequency N, from which may be determined the fluid's rheological properties.

15. A method as claimed in claim 14, using the pipe rheometer of any of claims 8, 9 and 10, and wherein the tank (13) is one of the pits containing the drilling fluid of a drilling rig.

**Patentansprüche**

1. Ein Rohr-Rheometer für das Messen der rheologischen Eigenschaften einer flüssigen Substanz, welches Rheometer von der Bauart ist mit einem Rohr (10), in welchem die Substanz zirkuliert, angetrieben von einer fluidbetätigten Membranpumpe (4) mit einer Fluideinlaßleitung (17), einer Fluidauslaßleitung (18), einem Pumpeneinlaß (19) und einem Pumpenauslaß (20), welcher Pumpeneinlaß die flüssige Substanz aufnimmt und welcher Pumpenauslaß mit einem Ende des Rohres (40) verbunden ist, <u>dadurch gekennzeichnet</u>, daß das andere Ende (21) des Rohres auf einem bekannten Druck gehalten wird, und ein Meßgerät (2) vorgesehen ist für das Messen des Druckes des die Pumpe (4) betätigenden Fluids, sowie Mittel (6,7) für das Bestimmen der Durchflußrate der flüssigen Substanz, die in dem Rohr (10) zirkuliert.

2. Das Rohr-Rheometer nach Anspruch 1, bei dem der bekannte Druck der Atmosphärendruck ist.

3. Das Rohr-Rheometer nach Anspruch 1 oder 2, ferner umfassend ein thermostatisches Bad (11), in welchem mindestens ein Teil des Rohres (10) eingeschlossen ist, um so das Fluid in dem Rohr innerhalb eines vorbestimmten Temperaturbereichs zu halten.

4. Das Rohr-Rheometer nach einem der Ansprüche 1 bis 3, bei dem die Mittel (6,7) für das Messen der Strömungsrate der flüssigen Substanz Mittel umfaßt für das Zählen der Anzahl von Pumpenzyklen während einer bestimmten Zeitperiode.

5. Das Rohr-Rheometer nach Anspruch 4, bei dem die Zählmittel ein Druckschalter (6), angeordnet in der Fluidauslaßleitung (18), und ein mit dem Druckschalter verbundener Zeitgeber (7) sind.

6. Das Rohr-Rheometer nach einem der vorangehenden Ansprüche, bei dem das Druckmeßgerät (2) sich in der Fluideinlaßleitung (17) befindet.

7. Das Rohr-Rheometer nach einem der vorangehenden Ansprüche, ferner umfassend Fluiddruckregulierungsmittel (2) in der Fluideinlaßleitung (17).

8. Das Rohr-Rheometer nach einem der vorangehenden Ansprüche, bei dem die flüssige Substanz in einem Tank (13) enthalten ist und das andere Ende (21) des Rohres (10) sich über der Oberfläche der flüssigen Substanz in dem Tank befindet, ferner umfassend eine Rücklaufleitung (15), die den Tank mit dem Pumpeneinlaß (19) verbindet.

9. Das Rohr-Rheometer nach Anspruch 8, ferner umfassend einen Mischer (16) in der Rücklaufleitung.

10. Das Rohr-Rheometer nach Anspruch 8 oder 9, ferner umfassend eine Rühreinrichtung (14) in dem Tank.

11. Das Rohr-Rheometer nach einem der vorangehenden Ansprüche, ferner umfassend eine erste Verbindungsleitung (9), angeschlossen zwischen dem Pumpenauslaß (20) und dem genannten einen Ende des Rohres (10), und/oder eine zweite Verbindungsleitung (12), befestigt an dem anderen Ende des Rohres (10), wobei die Geometrie der Verbindungsleitungen derart ist, daß der Druckabfall der flüssigen Substanz, die in ihnen zirkuliert, klein ist im Vergleich mit dem Druckabfall der flüssigen Substanz, die in dem Rohr zirkuliert.

12. Das Rohr-Rheometer nach einem der vorangehenden Ansprüche, bei dem das Druckmeßgerät (2) so geeicht ist, daß es der kleinen Differenz zwischen dem in der Fluideinlaßleitung (17) gemessenen Druck und dem Druck der flüssigen Substanz an dem Pumpenauslaß (20) Rechnung trägt.

13. Das Rohr-Rheometer nach einem der vorangehenden Ansprüche, bei dem das Fluid Druckluft ist.

**14.** Ein Verfahren zum Bestimmen der rheologischen Eigenschaften eines Bohrfluids, <u>dadurch gekennzeich-net</u>, daß ein Rohr-Rheometer nach einem der vorangehenden Ansprüche verwendet wird zum Messen des Fluidmeßdrucks P und der Pumpfrequenz N, woraus die rheologischen Eigenschaften des Fluids bestimmt werden können.

**15.** Ein Verfahren nach Anspruch 14 unter Verwendung des Rohr-Rheometers nach einem der Ansprüche 8, 9 und 10, und bei dem der Tank (13) einer der Sümpfe ist, welche die Bohrspülung eines Bohrrigs enthalten.

**Revendications**

**1.** Rhéomètre à conduit pour la mesure des propriétés rhéologiques d'une substance liquide, ledit rhéomètre étant du type comportant un conduit (10) dans lequel on fait circuler ladite substance, qui est propulsée par une pompe à diaphragme (4) entraînée par un fluide, comportant une conduite (17) d'entrée de fluide, une conduite (18) de sortie de fluide, une entrée de pompe (19) et une sortie de pompe (20), l'entrée de pompe recevant la substance liquide et la sortie de pompe étant connectée à une extrémité dudit conduit (40), **caractérisé en ce que** l'autre extrémité (21) du conduit est maintenue sous une pression connue, et en ce que l'on prévoit une jauge (2) pour mesurer la pression du fluide qui entraîne la pompe (4), ainsi que des moyens (6), (7) pour déterminer le débit de la substance liquide circulant dans le conduit (10).

**2.** Rhéomètre à conduit selon la revendication 1, **caractérisé en ce que** ladite pression connue consiste en la pression atmosphérique.

**3.** Rhéomètre à conduit selon la revendication 1 ou 2, comprenant de plus un bain thermostatique (11) dans lequel au moins une partie du conduit (10) est plongée, de manière à maintenir le fluide se trouvant dans le conduit dans un domaine prédéterminé de température.

**4.** Rhéomètre à conduit selon l'une quelconque des revendications 1 à 3, dans lequel les moyens (6, 7) pour mesurer le débit de la substance liquide comprennent des moyens pour compter le nombre de cycles de pompe sur une certaine période de temps.

**5.** Rhéomètre à conduit selon la revendication 4, dans lequel lesdits moyens de comptage consistent en un basculeur opérant sous l'action de la pression (6) (« pressure switch ») placé sur la ligne (18) de sortie de fluide, ainsi qu'en un dispositif de mesure du temps ou « timer » (7) connecté audit dispositif opérant sous l'action de la pression.

**6.** Rhéomètre à conduit selon l'une quelconque des revendications précédentes, dans lequel la jauge de pression (2) est placée sur la ligne (17) d'entrée de fluide.

**7.** Rhéomètre à conduit selon l'une quelconque des revendications précédentes, comprenant de plus des moyens de régulation (2) de la pression du fluide, disposés sur la ligne (17) d'entrée de fluide.

**8.** Rhéomètre à conduit selon l'une quelconque des revendications précédentes, dans lequel la substance liquide est contenue dans un réservoir (13) et l'autre extrémité (21) dudit conduit (10) est située au-dessus de la surface de la substance liquide dans ledit réservoir, et comprenant de plus une ligne de retour (15) reliant ledit réservoir avec l'entrée de pompe (19).

**9.** Rhéomètre à conduit selon la revendication 8, comprenant de plus un dispositif mélangeur (16) disposé sur la ligne de retour.

**10.** Rhéomètre à conduit selon la revendication 8 ou 9, comprenant de plus un dispositif d'agitation (14) placé dans le réservoir.

**11.** Rhéomètre à conduit selon l'une quelconque des revendications précédentes, comprenant de plus une première ligne (9) de connexion reliant la sortie de pompe (20) et ladite première extrémité du conduit (10) et/ou une seconde ligne (12) de connexion fixée à l'autre extrémité du conduit (10), la géométrie desdites lignes de connexion étant telle que la perte de charge de la substance liquide circulant dans ces

EP 0 458 391 B1

lignes est faible comparée à la perte de charge de la substance liquide circulant dans ledit conduit.

12. Rhéomètre à conduit selon l'une quelconque des revendications précédentes, dans lequel la jauge de pression (2) est calibrée de telle façon qu'elle tient compte de la légère différence existant entre la pression mesurée dans la ligne (17) d'entrée de fluide et la pression de la substance liquide à la sortie (20) de pompe.

13. Rhéomètre à conduit selon l'une quelconque des revendications précédentes, selon lequel le fluide consiste en air comprimé.

14. Procédé pour déterminer les propriétés rhéologiques d'un fluide de forage, **caractérisé en ce** que l'on utilise un rhéomètre à conduit selon l'une quelconque des revendications précédentes pour mesurer la pression P au niveau de la jauge concernant le fluide, ainsi que la fréquence N de la pompe, grandeurs à partir desquelles on peut déterminer les propriétés rhéologiques du fluide.

15. Procédé selon la revendication 14, utilisant le rhéomètre à conduit selon l'une quelconque des revendications 8, 9 et 10, et selon lequel le réservoir (13) consiste en l'un des bacs ou bassins à boue contenant le fluide de forage d'une installation de forage.

8

## Fig.1

## Fig.2

## Fig.3

| | |
|---|---|
| ☐ | ( 0.05 ) |
| ◆ | ( 0.1 ) |
| + | ( 0.15 ) |
| ◇ | ( 0.2 ) |
| ■ | ( 0.25 ) |
| ☐ | ( 0.3 ) |

## Fig.4

| | |
|---|---|
| ☐ | PIPE |
| ◇ | FANN |